# EUROPÄISCHE PATENTANMELDUNG

(11) **EP 2 082 722 A2**
(43) Veröffentlichungstag der Anmeldung: **29.07.2009**
(21) Anmeldenummer: 08020137.9
(22) Anmeldetag: 19.11.2008
(51) Int. Cl.: A61K 8/04, A61K 8/26, A61K 8/39, A61K 8/42, A61K 8/46, A61Q 19/10

(54) **Schäumbare selbsterwärmende kosmetische Zubereitungen**

(30) Priorität: 17.01.2008 DE 102008004862
(71) Anmelder: Beiersdorf AG, 20245 Hamburg (DE)
(72) Erfinder: Von Thaden, Stefanie, 20255 Hamburg (DE); Bauer, Anja, 22459 Hamburg (DE)
(74) Vertreter: Wilke, Jochen

(57) **Zusammenfassung**

Selbstwärmende kosmetische und/oder dermatologische Zubereitungen enthaltend
a) ein Tensidgemisch aus Monoisopropanolaminlaurethsulfat, Polyethylegiglycol(4)-Laurylether und Kokosfettsäurediethanolamid
b) einen oder mehrere Zeolite.
neben gegebenenfalls weiteren kosmetischen Wirk-, Hilfs- und Zusatzstoffen.

## Beschreibung

Die vorliegende Erfindung betrifft selbstwärmende kosmetische Zubereitungen.

Die vorliegende Erfindung betrifft kosmetische Reinigungsmittel. Derartige Mittel sind an sich bekannt. Es handelt sich dabei im wesentlichen um oberflächenaktive Substanzen oder Stoffgemische, die dem Verbraucher in verschiedenen Zubereitungen angeboten werden.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Die Forderungen an die Eigenschaften kosmetischer Reinigungspräparate haben sich in den letzten Jahren stark gewandelt. Früher standen Effekte wie Reinigen und Schäumen im Vordergrund der Verbraucherwünsche. Zur Zeit sind die ökologischen, ökonomischen und insbesondere dermatologischen Eigenschaften der Produkte vorrangig, obwohl das Schaumvermögen nach wie vor eine entscheidende Rolle spielt, beispielsweise als Indikator, um Restmengen von Tensiden nach der Reinigung von Haut und Haaren zu entfernen oder um Überdosierungen bei der Anwendung zu vermeiden. Allerdings steht bei kosmetischen Produkten - im Gegensatz zu den meisten technischen Reinigungsmitteln - die Haut- und Schleimhautverträglichkeit absolut im Vordergrund; die Produkte sollen "mild" sein.

Kosmetische oder dermatologische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen. Basis aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfemung und -lösung, ein leichtes Abspülen und - je nach Wunsch - auch für Schaumregulierung. Damit ist die Grundlage für die Schmutzentfernung lipidhaltiger Verschmutzungen gegeben.

Bei den hydrophilen Anteilen eines Tensidmoleküls handelt es sich meist um polare funktionelle Gruppen, beispielsweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische lonen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine lonen.

Die waschaktiven Tenside in kosmetischen und dermatologischen Reinigungsmitteln unterliegen einer sehr kritischen Beurteilung bezüglich ihres dermatologischen und ökologischen Verhaltens. Letzteres ist insbesondere deswegen von Bedeutung, da sie in erheblicher Menge angewendet werden und nach Gebrauch bestimmungsgemäß ins Abwasser gelangen.

Ausgehend von der bereits beschriebenen zentralen Bedeutung der waschaktiven Tenside für den Reinigungsvorgang ist ihr Verhalten auf der Humanhaut von größter Bedeutung.

Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut.

Es ist verständlich, dass waschaktive Tenside, die Haut und Haar von fettigen und wasserlöslichen Schmutzbestandteilen reinigen sollen, auch eine entfettende Wirkung auf die normalen Hautlipide haben. Bei jeder Hautreinigung werden in unterschiedlichem Maß auch interkorneozytäre Lipide und Sebumbestandteile entfernt. Das bedeutet, dass der natürliche Wasser-Lipid-Mantel der Haut bei jedem Waschvorgang mehr oder weniger gestört wird. Dies kann besonders bei extremer Entfettung zu einer kurzzeitigen Veränderung der Barrierefunktion der Haut führen, wobei selbstverständlich auch der jeweilige Zustand der behandelten Hautregion auf die dargestellten Veränderungen von erheblichem Einfluss ist. Beispielsweise kann die Hautdicke, die Anzahl der Talg- und Schweißdrüsen sowie die damit verbundene Empfindlichkeit erheblich variieren.

Grundsätzlich gilt dementsprechend als Forderung an waschaktive Tenside, dass sie biologisch möglichst inaktiv sind, um unerwünschte Nebenwirkungen zu vermeiden. Sie sollen ihre reinigende Wirkung bei optimaler Milde, bester Hautverträglichkeit und geringer Entfettung entfalten.

In der Regel sind kosmetische oder dermatologische Reinigungszubereitungen sehr gut auf ein angenommenes Anwendungsspektrum zugeschnitten, da für eine definierte, milde Reinigungswirkung insbesondere auch die je nach Anwendung unterschiedlichen mechanischen Parameter - wie beispielsweise der Zeitfaktor - von erheblicher Bedeutung sind: Dies wird z. B. deutlich, wenn man sich die unterschiedlichen Anwendungs- (Kontakt-) Zeiten eines Schaumbades im Vergleich zum kurzzeitigen Händewaschen vor Augen führt.

Um die reinigende Wirkung von Reinigungspräparaten zu erhöhen, können diese selbstwärmend ausgestaltet werden, das heißt mit Verbindungen, die unter Einwirkung von (Haut-) Feuchtigkeit bzw. Zugabe von Wasser Wärme erzeugen, eingesetzt. Der Vorteil dieser Methode besteht darin, dass unter Wärmeeinwirkung die Viskosität des Sebums abnimmt und sich dieses leichter entfernen lässt. Auch führt die Wärme zu einer Öffnung der Poren, so dass einerseits die Tiefenwirkung der Reinigung erhöht und andererseits das Eindringen von pflegenden Substanzen in die Haut erleichtert wird.

Der Nachteil des Standes der Technik von selbstwärmenden (oder besser selbst erwärmenden) Reinigungspräparaten besteht darin, dass ihre sensorischen Eigenschaften unbefriedigend sind. Zunächst lassen sich herkömmliche Zubereitungen von Masken schwer auf der Haut verteilen. Die Zubereitung wirkt klebrig und zäh. Nach erfolgter Anwendung wiederum lässt sich die Zubereitung nur schwer wieder von der Haut abspülen und entfernen.

Es war nun die Aufgabe der vorliegenden Erfindung, selbstwärmende kosmetische Zubereitungen zu entwickeln, die ein intensiveres Wärmegefühl hervorrufen, sich angenehmer auf der Haut anfühlen und besser auf der Haut zu verteilen sind.

Überraschenderweise wird die Aufgabe gelöst durch eine selbstwärmende kosmetische und/oder dermatologische Zubereitung enthaltend
a) ein Tensidgemisch aus Monoisopropanolaminlaurethsulfat (INCI: MIPA Laureth Sulfate), Polyethylenglycol(4)Laurylether (INCI: Laureth-4) und/oder Kokosfettsäurediethanolamid (INCI Cocamide DEA)
b) einen oder mehrere Zeolite,
neben gegebenenfalls weiteren kosmetischen Wirk-, Hilfs- und Zusatzstoffen.

Die erfindungsgemäßen Zubereitungen erwärmen sich deutlich bei Kontakt mit Wasser und zeichnen sich durch gute kosmetische Eigenschaften auf der Haut aus.

Erfindungsgemäß ist daher auch die Verwendung erfindungsgemäßer Zubereitungen als Wärme entwickelnde kosmetische Zubereitungen, dadurch gekennzeichnet, dass sie bei der kosmetischen Anwendung mit Feuchtigkeit zusammengeführt werden.

Insbesondere vorteilhaft ist es, die erfindungsgemäßen Zubereitungen als Reinigungszubereitungen auszugestalten.

Um eine größtmögliche Erwärmung zu erzielen, ist es daher von großem Vorteil, wenn die erfindungsgemäßen Zubereitungen selbst sich durch einen möglichst geringen Anteil an Wasser auszeichnen, im Idealfalle wasserfrei wären, bzw. allenfalls herstellungsbedingte Spuren von Wasser aufweisen.

Typischerweise beträgt der Restgehalt an Wasser in einer erfindungsgemäßen Zubereitung weniger als 0,1 Gewichts-%, bezogen auf das Gesamtgewicht der Zubereitung.

Überraschenderweise haben die erfindungsgemäßen Zubereitungen die Anmutung eines "Mousse", also eines angenehmen, reichhaltigen aber feinporigen Schaumes.

Es ist erfindungsgemäß vorteilhaft, die Gewichtsverhältnisse von Monoisopropanolaminlaurethsulfat zu Polyethylenglycol(4)Laurylether zu Kokosfettsäurediethanolamid wie a : b : c zu wählen, wobei a, b und c unabhängig voneinander rationale Zahlen von 1 bis 5, bevorzugt von 1 bis 3 darstellen können.

Insbesondere bevorzugt ist ein Gewichtsverhältnis von etwa 5 : 3 : 2, wie es etwa im käuflichen Tensidgemisch [Zetesol 100] von der Gesellschaft Zschimmer & Schwarz verwirklicht ist.

Es ist vorteilhaft im Sinne der vorliegenden Erfindung, die Gesamtmenge der Stoffe Monoisopropanolaminlaurethsulfat, Polyethylenglycol(4)Laurylether und Kokosfettsäurediethanolamid aus dem Bereich von 1 bis 20 Gew.-%, vorteilhaft von 2 bis 15 Gew.-%, insbesondere von 5 bis 10 Gew.-%, jeweils bezogen auf das Gesamtgewicht der Formulierung, zu wählen.

Es ist erfindungsgemäß vorteilhaft, wenn die erfindungsgemäße Zubereitung Zeolite in einer Menge von 10 bis 50 Gewichts-% und besonders bevorzugt 20 bis 40 Gewichts-% jeweils bezogen auf das Gesamtgewicht der Zubereitung enthält.

Dabei ist es erfindungsgemäß von Vorteil, wenn als Zeolite Natriumaluminiumsilikate und Natriumkaliumaluminiumsilikate in Pulverform wie Molecular Sieve Type GMP-3A Powder (UOP), Molecular Sieve Type GMP-4A Powder (UOP), Molekularsieb 0,5 nm (Merck), Valfor Zeolite Na-A (The PQ Corporation) oder Zeolite von Honeywell Specialty Chemicals und der Sithean Corporation eingesetzt werden.

Insbesondere bevorzugt ist die Verwendung von Molekularsieb 0,5 nm.

Die erfindungsgemäße Zubereitung weist nach dem Zusatz von Wasser erfindungsgemäß vorteilhaft einen pH-Wert von pH 5 bis pH 8 auf. Erfindungsgemäß bevorzugt weist die erfindungsgemäße Zubereitung bei der Anwendung einen pH-Wert von pH 6,5 bis pH 8 auf.

Zur Einstellung des pH-Wertes ist es erfindungsgemäß vorteilhaft, wenn die erfindungsgemäße Zubereitung eine oder mehrere Säuren in einer Menge von 0,1 bis 3 Gewichts-% und besonders bevorzugt 0,8 bis 1,5 Gewichts-% jeweils bezogen auf das Gesamtgewicht der Zubereitung enthält.

Dabei ist es erfindungsgemäß von Vorteil, wenn die Säuren gewählt werden aus der Gruppe der anorganischen Säuren (verdünnte Salzsäure, verdünnte Phosphorsäure, verdünnte Essigsäure) und der organischen Säuren (Milchsäure, Zitronensäure, Ascorbinsäure, Salicylsäure, Weinsäure, Bernsteinsäure, Adipinsäure, Malonsäure), besonders vorteilhaft Milchsäure und Zitronensäure. Dabei sind Milchsäure und Zitronensäure erfindungsgemäß bevorzugt.

Die kosmetischen und dermatologischen Zubereitungen gemäß der Erfindung können kosmetische Hilfsstoffe enthalten, wie sie üblicherweise in solchen Zubereitungen verwendet werden, z.B. Parfüme, Verdickungsmittel, Wirkstoffe, Konditionierer oder andere übliche Bestandteile einer kosmetischen oder dermatologischen Formulierung wie Alkohole, Polymere, Elektrolyte, organische Lösungsmittel oder Silikonderivate. Ferner können sie Abrasiva enthalten.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **Beispiel Nr.** | | | | | | | | | |
|---|---|---|---|---|---|---|---|---|---|---|
| | **1** | **2** | **3** | **4** | **5** | **6** | **7** | **8** | **9** | **10** |
| Wasser | 0,06 | 0,06 | 0,06 | 0,06 | 0,06 | 0,06 | 0,06 | 0,06 | 0,06 | 0,06 |
| Zitronensäure | 0,18 | 0,18 | 0,18 | 0,18 | 0,18 | 0,18 | 0,18 | 0,18 | 0,18 | 0,12 |
| Dibuthylhydroxytoluol | 0,05 | 0,05 | 0,05 | 0,05 | 0,05 | 0,05 | 0,05 | 0,05 | 0,05 | 0,05 |
| Parfüm | 1 | 1 | 1 | 1 | 1 | 1 | 0 | 1 | 1 | 1 |
| Monoisopropanolaminlaurethsulfat, Polyethylenglycol(4)Lauryl-ether + Kokosfettsäurediethanolamid | 13,5 | 13,5 | 13,5 | 13,5 | 13,5 | 0 | 0 | 0 | 0 | 0 |
| Monoisopropanolaminlaurethsulfat, Polyethylenglycol(4)Lauryl-ether und Propylenglycol | 0 | 0 | 0 | 0 | 0 | 13,5 | 13,5 | 13,5 | 13,5 | 13,5 |
| (Poly(oxyethylene)m-block-poly(oxypropylene)n-block-poly(oxyethylene)p) | 2 | 2 | 2 | 2 | 2 | 2 | 2 | 2 | 2 | 2 |
| Rizinusöl | 14 | 18 | 14 | 14 | 14 | 14 | 14 | 14 | 14 | 14 |
| Weizenkeimöl | 0 | 0 | 0 | 0 | 0 | 0 | 0 | 0 | 0 | 0,2 |
| Molekularsieb 0,5 nm | 35 | 25 | 40 | 35 | 35 | 35 | 35 | 35 | 35 | 35 |
| Magnesiumsulfat wasserfrei | 15 | 20 | 10 | 15 | 15 | 15 | 15 | 15 | 15 | 15 |
| Propylencarbonat | 0 | 0 | 0 | 0,99 | 0 | 0,6 | 0 | 0 | 0 | 0 |
| Sojaöl + Rizinusöl + Propyl-3,4,5-trihydroxybenzoat + Zitronensäure | ad 100 | ad 100 | ad 100 | ad 100 | ad 100 | ad 100 | ad 104 | ad 100 | ad 100 | ad 100 |

## Patentansprüche

1. Selbstwärmende kosmetische und/oder dermatologische Zubereitungen enthaltend
a) ein Tensidgemisch aus Monoisopropanoiaminlaurethsulfat, Polyethylenglycol(4)-Laurylether und Kokosfettsäurediethanolamid
b) einen oder mehrere Zeolite,
neben gegebenenfalls weiteren kosmetischen Wirk-, Hilfs- und Zusatzstoffen.

2. Zubereitungen nach Anspruch 1, in denen die Gewichtsverhältnisse von Monoisopropanolaminlaurethsulfat zu Polyethylenglycol(4)Laurylether zu Kokosfettsäurediethanolamid wie a : b : c gewählt werden, wobei a, b und c unabhängig voneinander rationale Zahlen von 1 bis 5, bevorzugt von 1 bis 3 darstellen.

3. Zubereitungen nach Anspruch 2, durch ein Gewichtsverhältnis von von Monoisopropanolaminlaurethsulfat zu Polyethylenglycol(4)Laurylether zu Kokosfettsäurediethanolamid wie etwa 5 : 3 : 2 gewählt wird.

4. Zubereitungen nach einem der vorstehenden Ansprüche, **gekennzeichnet durch** eine Gesamtmenge der Stoffe Monoisopropanolaminlaurethsulfat, Polyethylenglycol(4)Lauryl-ether und Kokosfettsäurediethanolamid gewählt aus dem Bereich von 1 bis 20 Gew.-%, vorteilhaft von 2 bis 15 Gew.-%, insbesondere von 5 bis 10 Gew.-%, jeweils bezogen auf das Gesamtgewicht der Formulierung.

5. Zubereitungen nach einem der vorstehenden Ansprüche, **gekennzeichnet durch** einen Gehalt von 10 bis 50 Gewichts-% an einem oder mehreren Zeoliten, besonders bevorzugt 20 bis 40 Gewichts-% jeweils bezogen auf das Gesamtgewicht der Zubereitung.

6. Zubereitungen nach einem der vorstehenden Ansprüche, **gekennzeichnet** dass der oder die Zeolite gewählt wird oder werden aus der Gruppe der Natriumaluminiumsilikate und Natriumkaliumaluminiumsilikate in Pulverform wie Molecular Sieve Type GMP-3A Powder (UOP), Molecular Sieve Type GMP-4A Powder (UOP), Molekularsieb 0,5 nm, Valfor Zeolite Na-A.

7. Zubereitungen nach einem der vorstehenden Ansprüche, **dadurch gekennzeichnet, dass** als Zeolite das Molekularsieb 0,5 nm gewählt wird.

8. Zubereitungen nach einem der vorstehenden Ansprüche, durch einen Wassergehalt von weniger als 0.1Gewichts-% **gekennzeichnet**, bezogen auf das Gesamtgewicht der Zubereitung.

9. Verwendung von Zubereitungen nach einem der vorstehenden Ansprüche als wärmeentwickelnde kosmetische Zubereitungen, **dadurch gekennzeichnet, dass** sie bei der kosmetischen Anwendung mit Feuchtigkeit zusammengeführt werden.

10. Verwendung von Zubereitungen nach einem der vorstehenden Ansprüche als wärmeentwickelnde Reinigungszubereitungen.
